# EUROPÄISCHE PATENTSCHRIFT

(11) **EP 0 907 348 B1**
(45) Veröffentlichungstag und Bekanntmachung des Hinweises auf die Patenterteilung: **17.07.2002**
(21) Anmeldenummer: 97921836.9
(22) Anmeldetag: 30.04.1997
(51) Int. Cl.: A61K 7/16

(54) **ZAHNPUTZMITTEL ALS GRANULAT**
TOOTH-CLEANING AGENT IN GRANULAR FORM
PRODUIT POUR LE NETTOYAGE DES DENTS SOUS FORME DE GRANULES

(30) Priorität: 03.05.1996 DE 19617723
(43) Veröffentlichungstag der Anmeldung: 14.04.1999
(73) Patentinhaber: Private Universität Witten/Herdecke GmbH, 58448 Witten (DE)
(72) Erfinder: GÄNGLER, Peter, D-58448 Witten (DE)
(74) Vertreter: Meyers, Hans-Wilhelm, Dr.Dipl.-Chem.
(86) Internationale Anmeldenummer: EP9702218
(87) Internationale Veröffentlichungsnummer: WO9741829

(56) Entgegenhaltungen:
- WO-A-94/22417
- BE-A- 562 960
- US-A- 5 094 839

## Beschreibung

Der Gegenstand der vorliegenden Erfindung ist ein Zahnputzmittel mit anorganischen und/oder organischen Partikeln, eine Vorrichtung zur Applikation des Zahnputzmittels sowie eine Zusammenstellung enthaltend das Zahnputzmittel und die Vorrichtung gemäß der Erfindung.

Die klassische systematische Mundhygiene, welche vor ca. 100 Jahren eingeführt wurde, beruht im wesentlichen auf Zahncremes, die mit Zahnbürsten appliziert werden sowie Mundwässern. Diese Mittel haben bereits einen hohen hygienischen Standard ermöglicht.

JP-01299-211-A beschreibt ein Zahnputzmittel bestehend aus einem Granulat erhältlich aus einem wasserunlöslichen Pulver und einem wasserunlöslichen anorganischen Bindemittel mit einer Korngröße, die durch ein 30 mesh-Sieb aber nicht durch ein 200 mesh-Sieb paßt. Das Granulat zerfällt unter einer Belastung von 0,1 bis 10 g pro Partikel. Das Pulver hat bevorzugterweise eine Korngröße von 0,1 bis 20 µm. Das Granulat ist bevorzugterweise farbig und im wesentlichen kugelförmig und wird vorzugsweise durch Sprühgranulierung hergestellt. Weiter werden verschiedene anorganische Verbindungen für Pulver und Bindemittel vorgeschlagen. Das Granulat wird mit 50 bis 99 Gew.-% weiterer Bestandteile vermischt.

JP-04243816-A beschreibt ein Granulat, das zwei oder mehr Arten von Partikeln mit einer Belastbarkeit von 0,1 bis 50 g pro Partikel beschreibt, wobei sich die beiden Arten in der Belastbarkeit unterscheiden. Das Mittel beinhaltet a) Granulat mit einer mittleren Korngröße von 100 bis 500 µm und einer Belastbarkeit von 0,1 bis 10 g pro Partikel und b) Granulat mit einer durchschnittlichen Korngröße von 20 bis 200 µm und einer Belastbarkeit von 10 bis 50 g pro Partikel mit einem Gewichtsverhältnis a) zu b) von 0,1 bis 20. Das Granulat wird mit 50 bis 99 % weiterer Bestandteile vermischt.

US 5,496,541 beschreibt Zahnputzmittel mit einer Dreikomponentenmischung von Detergenzien mit einer verbesserten Schaumbildung, die unter anderem auch in Granulatform eingesetzt werden kann.

Auf Grundlage neuer wissenschaftlicher Erkenntnisse der letzten Jahrzehnte erscheint es notwendig, Zahnputzmittel in einer Vielzahl verschiedener Darreichungsformen, insbesondere mit einem hohen Grad an Individualisierungsmöglichkeiten und Abstimmung auf einzelne Risikofaktoren über verschiedene Spannen des Lebenszeitraums hinweg bereitzustellen. Die bislang erhältlichen Zahnputzmittel sind nur unter unvertretbar hohem wirtschaftlichen Aufwand in der Lage, solche Bedürfnisse zu befriedigen. Erforderlich ist daher ein neues Zahnpflegesystem, was den angesprochenen Anforderungen hinsichtlich der Individualisierung und Berücksichtigung neuer wissenschaftlicher Erkenntnisse entspricht.

So ist es Sinn einer an natürlichen Bedürfnissen orientierten Mundhygiene, die Zähne, das Zahnfleisch, das Zahnbett und die Mundschleimhaut zu schützen und zu schonen.

Das der Erfindung zugrundeliegende Problem wird gelöst durch ein Zahnputzmittel gemäß Patentanspruch 1. Die Ansprüche 2 bis 7 betreffen bevorzugte Ausführungsformen des erfindungsgemäßen Zahnputzmittels.

Das erfindungsgemäße Zahnputzmittel beinhaltet mindestens zwei Darreichungsformen.

Mit Hilfe des erfindungsgemäßen Zahnputzmittels ist es in sehr einfacher Weise möglich, unterschiedliche Eigenschaften, z.B. die Abrasivität des Zahnputzmittels zu steuern. Ein Zahnputzmittel geringerer Abrasivität wird beispielsweise über sechs Tage angewendet und am siebten Tag wird ein Granulat mit höherer Abrasivität verwendet, wobei das Granulat mit höherer Abrasivität eine Revelatorsubstanz zur Kontrolle der Entfernung organischer Oberflächenschichten auf den Zähnen beinhaltet. Diese Darreichungsform hat den Vorteil, daß Zahnbeläge effektiver mit einer Zahncreme höherer Abrasivität entfernt werden können, wohingegen die normale Zahnpflege über einen längeren Zeitraum auf Zahnputzmittel hoher Abrasivität verzichtet. Hohe Abrasivität des Zahnputzmittels hat bei zu intensiver Anwendung den gravierenden Nachteil, daß Putzdefekte am Zahnschmelz auftreten können.

Das erfindungsgemäße Zahnputzmittel weist anorganische und/oder organische Materialien auf, die als Granulat vorliegen, wobei das Granulat eine mittlere Teilchengröße von 10 bis 500 µm aufweist. Dem erfindungsgemäßen Granulat sind gegebenenfalls weitere Träger- und/oder Hilfsstoffe zumischbar. Das im Granulat enthaltene die Abrasivität bestimmende Material hat eine mittlere Teilchengröße von 0,04 bis 1,20 µm.

Durch die erfindungsgemäße Darreichungsform kann mithin eine optimale Zahnpflege sowohl unter medizinischen wie auch kosmetischen Gesichtspunkten erreicht werden.

Das erfindungsgemäße Zahnputzgranulat ist vorteilhaft, da keine Konservierungsstoffe verwendet werden müssen. Dem erfindungsgemäßen Zahnputzmittel muß darüber hinaus kein Bindemittel und kein Feuchtmittel beigefügt werden, so daß sich das Gewicht der entsprechenden Zahnputzmittel erheblich verringert. Dies hat weitere Vorteile hinsichtlich der Distribution der entsprechenden Produkte zur Folge. Desweiteren kann die Tensidmenge verringert werden, die in konventionellen Zahncremes verbreitet ist. Da die erfindungsgemäßen Zahnputzmittel in trockener Form vorliegen, sind sie gegen Austrocknen unempfindlich und können somit länger gelagert werden, auch unter extremen klimatischen Bedingungen. Vorzugsweise wird das Granulat aus einem Pulver hergestellt. Die Herstellung von Granulaten ist an sich bekannt. Vorzugsweise besteht das zu granulierende Material aus einem Pulver, wie mikrokristalline Cellulose, Carboxymethylcellulose, Hydroxyethylcellulose, Calciumhydrogenphosphat, Calciumcarbonat oder Siliciumdioxid.

Als Wirkstoffe kommen insbesondere organische und/oder anorganische fluoridhaltige Substanzen, Pyrophosphate, Revelatorsubstanzen, wie Erythrosin oder Patentblau, calciumphosphathaltige Substanzen, Speichelstimulantien, Partikel bestimmter Abrasivität und/oder Tenside in Frage.

Darüber hinaus können Hilfsstoffe wie Duft-, Geschmacks- und/oder Farbstoffe eingesetzt werden.

Die relativen Mengen umfassen zum Beispiel:
0,24 - 0,32 Massen-% Natriumfluorid (entspricht 1.100 bis 1.450 ppm F⁻) als Kariesschutz und zur Remineralisation früher Läsionen, 3 - 4 Massen-% Tetranatriumpyrophosphat als Zahnsteinbildungshemmer, 0,1 Massen-% Erythrosin als Revelator zum Anfärben von weichen Zahnbelägen (Plaque). Desweiteren können calciumphosphathaltige Substanzen zur Förderung der Remineralisation des Zahnschmelzes und des Zahnbeins an Zahnwurzeln und Füllungsrändern als Bestandteil des Grundpulvers zugemischt sein, neben Vitamin C, eingestellt auf einen pH 5 - 6 als Mittel zur Speichelstimulation, besonders bei sehr häufigen Speichelsekretionsstörungen. Dieser Zusatz dient gleichzeitig zur Förderung der Remineralisation des Zahnschmelzes und des Zahnbeins. Gegebenenfalls sind Geschmacks- und Duftstoffe je nach Zeitgeschmack, Altersgruppe und Risikogruppe zumischbar.

0,5 - 1,2 Massen-% Tenside können zur Verstärkung des Frischegefühls, je nach Altersgruppe beigefügt sein, aber ingesamt reduziert gegenüber der Anwendung in konventionellen Zahnpasten.

Vorteilhaft ist der Einsatz von Vitamin C zur gleichzeitigen Speichelstimulation, zur Förderung der Remineralisation durch oberflächlichen Angriff an Zahnschmelz und Zahnbein und vermehrten Speichelfluß und zur potentiellen Modulation der lokalen Immunreaktion als Radikalfänger (Entzündungshemmung).

Die Individualisierung berücksichtigt Risikofaktoren der Mundgesundheit, wie Kariesrisiko, Parodontitisrisiko, Zahnsteinbildung, Speichelsekretionsstörungen in den verschiedenen Altergruppen und Erkrankungsgruppen und kann demzufolge unterschiedliche Formulierungen des Granulates umfassen:
Zahnputzgranulat für Kinder mit geringer Abrasivität, Fluoridkonzentration von ca. 1.100 ppm Fluorid,
Zahnputzgranulat für Jugendliche mit geringer Abrasivität, Fluoridkonzentration von ca. 1.450 ppm, eventuell bekannten Substanzen der chemischen Plaquehemmung,
Zahnputzgranulat für Erwachsene mit mittlerer Abrasivität, Fluoridkonzentration von ca. 1.450 ppm sowie Substanzen zur Zahnsteinbildungshemmung, z.B. Tetranatriumpyrophosphat oder
Zahnputzgranulat bei Speichelsekretionsstörungen, z.B. durch systemische Medikamenteneinwirkungen, mit Zusätzen von Vitamin C, pH 5 - 6 zur Förderung des Speichelflusses und mit der vorteilhaften Doppelwirkung bei der Schmelzremineralisation.

Als weitere Formulierungsvarianten sind naturheilkundliche Zahnputzgranulate, z.B. mit Pflanzenextrakten sowie homöopathische Zahnputzgranulate, z.B. mit homöopathischen Auszügen, von praktischer Bedeutung. Vorteilhafterweise können diese Wirkstoffe auch nachträglich einem Granulat zugesetzt werden.

Bevorzugt ist eine rasche Löslichkeit des Granulats sowie dessen Fähigkeit, sogenannte Slow-Release-Substanzen zu inkorporieren. Als Slow-Release-Substanzen kommen insbesondere biodegradierbare Hydrogele (Aerogele) bzw. alternative Polymere in Frage.

Eine Zahnbürste zur Applikation des Zahnputzmittels besteht vorteilhafterweise aus einem inneren Borstenbüschelfeld, das von einem äußeren Borstenbüschelsaum überragt wird. Die Borstenbüschel im inneren Feld sind so angeordnet, daß das Granulat nicht zwischen ihnen hindurchfallen kann. Die durch die Borsten gebildete muldenartige Vertiefung sollte dabei dem Volumen des Zahnputzmittels für einmalige Anwendung entsprechen.

Die unterschiedliche Höhe der Borstenbüschel gewährleistet die Applikation des Zahnputzmittels und dient gleichzeitig einer besseren mechanischen Zahnreinigung in den Zahnzwischenräumen. Grundsätzlich ist es jedoch auch möglich, das Granulat auf einer herkömmlichen angefeuchteten Zahnbürste anzuordnen und damit die Mundhygiene auszuführen.

In vorteilhafter Weise wird beispielsweise das Zahnputzgranulat nach einem Konzept 6 + 1 in zwei unterschiedlichen Farben in einem Spender dem Konsumenten gereicht. Nach diesem Konzept besteht die größere Menge, die in einem Teil des Spenders angeordnet ist, aus Zahnputzmittel für beispielsweise 6 Tage. Dieses Zahnputzmittel besteht aus einem Granulat niedriger Abrasivität mit Fluoriden, Pyrophosphat, eventuell Speichelstimulantien. Am siebten Tag wird ein Granulat mit höherer Abrasivität, das einen Revelator enthält, eingesetzt. Durch die höhere Abrasivität werden die mit dem Revelator gleichzeitig angefärbten Zahnbeläge kontrollierbar und gründlich entfernt.

In einer einfachen Ausführungsform des Spenders besteht dieser aus einer Vorrichtung mit zwei Kammern mit unterschiedlicher Kapazität, in welchem das Granulat abgefüllt ist. Dieses kann durch eine Entnahmeeinrichtung entnommen werden und auf eine Zahnbürste zur Applikation des Zahnputzmittels verbracht werden.

Vorzugsweise entspricht das Verbrauchsvolumen des Spenders der Grenznutzungsdauer der Borstenbüschel der erfindungsgemäßen Zahnbürste.

Durch die erfindungsgemäße Darreichungsform des erfindungsgemäßen Zahnputzgranulats ist es möglich, in besonders einfacher und effektiver Weise Mundhygienemittel, insbesondere Zahnputzmittel bereitzustellen, die den speziellen Bedürfnissen von Kindern (Milchgebiß), Jugendlichen und Erwachsenen dienen. Die Verwendung von Speichelstimulantien ist für die bekannten großen Risikogruppen vorteilhaft. Als Risikogruppen sind insbesondere Personen mit Mundtrockenheit und Speichelsekretionsstörungen zu nennen. Solche Störungen können durch blutdrucksenkende Medikamente, Psychopharmaka und Bestrahlung ausgelöst werden. Auch Raucher bilden eine bekannte Risikogruppe. Es ist ebenfalls möglich, individualisierte Sets bzw. Spender anzubieten als allopathisches Zahnputzgranulat, naturheilkundliches Zahnputzgranulat, homöopathisches Zahnputzgranulat und Zahnputzgranulat mit gesteuerter Abrasivität.

In vorteilhafter Weise kann die Vorrichtung zur Applikation des Zahnputzmittels oder eine konventionelle Zahnbürste zusammen mit der Darreichungsform angeboten werden. Dadurch hat der Konsument die Möglichkeit, genau festzustellen, wann die Zahnbürste gewechselt werden soll, nämlich dann, wenn das Zahnputzgranulat aufgebraucht ist.

Das durch die erfindungsgemäßen Mittel, Zahnputzmittel, Vorrichtung zur Applikation des Zahnputzmittels und Zusammenstellung enthaltend ein Zahnputzmittel bereitgestellte neue Zahnpflegesystem liefert eine völlig neue Motivation zur Mundhygiene. Nebenwirkungen und Risikozusatzstoffe, die in konventionellen Zahncremes nicht zu vermeiden sind, können vollständig eliminiert werden. Zahnputzschäden, die eine zunehmend klinisch relevante Rolle spielen, werden auf ein Minimum reduziert. Neue Erkenntnisse aus Wissenschaft und Zahnheilkunde betreffend Aspekte der Mundhygiene könnten in Zukunft rascher realisiert werden. Auf der Grundlage einer natürlichen Zahnpflege und Mundhygiene wird die Remineralisation, die antiphlogistische Wirkung, die Reduktion von Plaque und von Zahnstein optimiert.

### Beispiel für die Formulierung eines erfindungsgemäßen Zahnputzgranulates

Calciumcarbonat, Calciumhydrogenphosphat und Siliciumdioxid als Grundpulver mit Zusatzsubstanzen
1.450 ppm F⁻ Natriumfluorid
3 - 4 Massen-% Tetranatriumpyrophosphat
1 - 2 Massen-% Vitamin C pH 5
Geschmacks- und Duftstoffe in Spuren
0,7 Massen-% Tenside, wie Natriumlaurylsulfat Farbe, z.B. blau, durch Lebensmittelfarbstoff (für 6 Tage).

Nach einem Konzept 6 + 1 ändert sich das Beispiel der Formulierung des Zahnputzmittels für den 7. Tag durch erhöhte Abrasivität. Diese kann durch einen höheren Anteil an Siliciumdioxid eingestellt werden. Durch Zugabe eines Revelators mit Erythrosin wird vorhandene Plaque rot eingefärbt. Der Tensidgehalt ist verringert oder eliminiert. Die Besonderheit des Zahnputzmittels besteht darin, daß nach freier Wahl die organischen Zahnbeläge gleichzeitig eingefärbt und durch den verringerten oder ganz eliminierten Tensidgehalt gut kontrollierbar gründlich entfernt werden können.

## Patentansprüche

1. Zahnputzmittel in Granulatform aus anorganischem und/oder organischem Material sowie gegebenenfalls weiteren Träger- und/oder Hilfsstoffen, **dadurch gekennzeichnet, daß**
- das Zahnputzmittel getrennt in mindestens zwei verschiedenen Darreichungsformen vorliegt, die sich in ihrer Wirkstoffzusammensetzung unterscheiden.

2. Zahnputzmittel nach Anspruch 1, **dadurch gekennzeichnet, daß** das Granulat als Wirkstoffe organische und/oder anorganische fluoridhaltige Substanzen, Pyrophosphate, Revelatorsubstanzen, calciumphosphathaltige Substanzen, Speichelstimulantien, Tenside und/oder Partikel bestimmter Abrasivität enthält.

3. Zahnputzmittel nach Anspruch 1 und/oder 2, **dadurch gekennzeichnet, daß** das Granulat aus einem Pulver hergestellt worden ist.

4. Zahnputzmittel nach mindestens einem der Ansprüche 1 bis 3, **dadurch gekennzeichnet, daß** das Pulver aus mikrokristalliner Cellulose, Carboxymethylcellulose, Hydroxyethylcellulose, Calciumhydrogenphosphat, Calciumcarbonat oder Siliciumdioxid besteht.

5. Zahnputzmittel nach mindestens einem der Ansprüche 1 bis 4, **dadurch gekennzeichnet, daß** die mittlere Teilchengröße des die Abrasivität bestimmenden Materials 0,04 bis 1,20 µm beträgt und in einem Granulat enthalten ist, das eine mittlere Teilchengröße von 10 bis 500 µm aufweist.

6. Zahnputzmittel nach mindestens einem der Ansprüche 1 bis 5, **dadurch gekennzeichnet, daß** das Granulat weitere Hilfsstoffe wie Duft-, Geschmacks- und/oder Farbstoffe enthält.

7. Zahnputzmittel nach einem der Ansprüche 1 bis 6, **dadurch gekennzeichnet, daß** durch den nachträglichen Zusatz weiterer Stoffe eine Individualisierung des Zahnputzmittels für den Anwender erreicht wird.

8. Zahnbürste mit Haltegriff und Kopfbereich zur Applikation des Zahnputzmittels nach mindestens einem der Ansprüche 1 bis 7, **dadurch gekennzeichnet, daß** im Kopfbereich ein inneres Borstenbüschelfeld von einem äußeren Borstenbüschelsaum überragt wird.

9. Zusammenstellung enthaltend ein Zahnputzmittel nach mindestens einem der Ansprüche 1 bis 7 und eine Zahnbürste, insbesondere eine Vorrichtung nach Anspruch 8, wobei das Verbrauchsvolumen des Zahnputzsmittels der Grenznutzungsdauer der Borstenbüschel entspricht.

10. Zusammenstellung nach Anspruch 9 **dadurch gekennzeichnet, daß** das Zahnputzmittel in zwei Darreichungsformen enthalten ist, wobei die Menge der ersten Darreichungsform etwa das sechsfache der zweiten Darreichungsform beträgt und die Abrasivität der ersten Darreichungsform geringer ist als diejenige der zweiten Darreichungsform.

11. Zusammenstellung nach Anspruch 10, **dadurch gekennzeichnet, daß** das Granulat mit höherer Abrasivität eine Revelatorsubstanz zur Kontrolle der Entfernung organischer Oberflächenschichten auf den Zähnen beinhaltet.

## Claims

1. A dentifrice in the form of granules consisting of an inorganic and/or organic material and optionally further carriers and/or auxiliary agents, **characterized in that**
- said dentifrice is present in at least two separate distinct dosage forms which are distinguished by their compositions of active substances.

2. The dentifrice according to claim 1, **characterized in that** said granules contain organic and/or inorganic fluoride-containing substances, pyrophosphates, revelator substances, substances containing calcium phosphate, saliva stimulants, surfactants and/or particles of defined abrasivity as active substances.

3. The dentifrice according to claim 1 and/or 2, **characterized in that** said granules has been prepared from a powder.

4. The dentifrice according to at least one of claims 1 to 3, **characterized in that** said powder consists of microcrystalline cellulose, carboxymethylcellulose, hydroxyethylcellulose, calcium hydrogenphosphate, calcium carbonate or silicon dioxide.

5. The dentifrice according to at least one of claims 1 to 4, **characterized in that** the average particle size of the abrasivity-determining material is from 0.04 to 1.20 µm, contained in granules having an average particle size of from 10 to 500 µm.

6. The dentifrice according to at least one of claims 1 to 5, **characterized in that** said granules contain additional auxiliaries, such as perfumes, flavors and/or colorants.

7. The dentifrice according to at least one of claims 1 to 6, **characterized in that** individualization of the dentifrice for the user is achieved by the later addition of further ingredients.

8. A toothbrush comprising a gripping handle and head region for the application of the dentifrice according to at least one of claims 1 to 7, **characterized in that** an inner bristle bunch field is overtopped by an outer bristle bunch skirt.

9. A kit containing a dentifrice according to at least one of claims 1 to 7 and a toothbrush, especially a device according to claim 8, wherein the consumable volume of the dentifrice corresponds to the maximum admissible service life of the bristle bunches.

10. The kit according to claim 9, **characterized in that** said dentifrice is contained in two dosage forms, wherein the quantity of the first dosage form is about six times the quantity of the second dosage form, and the abrasivity of the first dosage form is lower than that of the second dosage form.

11. The kit according to claim 10, **characterized in that** the granules having a higher abrasivity comprise a revelator substance for checking the removal or organic surface layers on the teeth.

## Revendications

1. Abrasif dentaire sous forme de granulés constitués de matière minérale et/ou organique ainsi qu'éventuellement d'autres substrats et/ou adjuvants, **caractérisé en ce que**
- l'abrasif dentaire est présenté sous une forme divisée en au moins deux formes posologiques différentes qui se différencient dans leur composition en substances actives.

2. Abrasif dentaire selon la revendication 1, **caractérisé en ce que** les granulés contiennent, en tant que substances actives, des composés fluorés organiques et/ou minéraux, des pyrophosphates, des composés révélateurs, des composés contenant du phosphate de calcium, des agents salivants, des tensioactifs et/ou des particules d'une abrasivité déterminée.

3. Abrasif dentaire selon la revendication 1 et/ou 2, **caractérisé en ce que** les granulés sont préparés à partir d'une poudre.

4. Abrasif dentaire selon une des revendications de 1 à 3, **caractérisé en ce que** la poudre se compose de cellulose microcristalline, de carboxyméthylcelllulose, d'hydroxyéthylcellulose, d'hydrogénophosphate de calcium, de carbonate de calcium, ou de dioxyde de silicium.

5. Abrasif dentaire selon au moins une des revendications de 1 à 4, **caractérisé en ce que** la granulométrie moyenne de la matière déterminant l'abrasivité est de 0,04 à 1,20 µm et que cette matière est contenue dans des granulés ayant une granulométrie moyenne de 10 à 500 µm.

6. Abrasif dentaire selon au moins une des revendications de 1 à 5 , **caractérisé en ce que** les granulés contiennent d'autres adjuvants tels que des agents odorants, des agents gustatifs et/ou des colorants.

7. Abrasif dentaire selon une des revendications de 1 à 6, **caractérisé en ce que** l'on obtient une individualisation de l'abrasif dentaire pour l'utilisateur en ajoutant ultérieurement d'autres substances.

8. Brosse à dents comportant une poignée de maintien et une zone de tête destinée à appliquer l'abrasif dentaire selon une des revendications de 1 à 7, **caractérisée en ce que**, dans la zone de tête, une touffe de poils de porc extérieure dépasse en hauteur une touffe de poils de porc intérieurs.

9. Ensemble comportant un abrasif dentaire selon au moins une des revendications de 1 à 7 et une brosse à dents, notamment un appareil selon la revendication 8, pour lequel le volume de consommation de l'abrasif dentaire correspond à la durée limite d'utilisation des touffes de poils de porc.

10. Ensemble selon la revendication 9, **caractérisé en ce que** l'abrasif dentaire est contenu sous deux formes posologiques, dans lequel la quantité de la première forme posologique est environ six fois supérieure à celle de la seconde forme posologique et l'abrasivité de la première forme posologique est plus faible que celle de la seconde forme posologique.

11. Ensemble selon la revendication 10, **caractérisé en ce que** les granulés ayant une abrasivité supérieure contiennent un révélateur destiné à contrôler l'élimination des couches superficielles organiques sur les dents.
